# FASCICULE DE BREVET EUROPEEN

(11) **EP 1 148 871 B1**
(45) Date de publication et mention de la délivrance du brevet: **01.06.2005**
(21) Numéro de dépôt: 99947592.4
(22) Date de dépôt: 15.10.1999
(51) Int. Cl.: A61K 9/20, A61K 31/64

(54) **COMPRIME MATRICIEL PERMETTANT LA LIBERATION PROLONGEE DE GLICLAZIDE APRES ADMINISTRATION PAR VOIE ORALE**
GLICAZIDHALTIGE MATRIXTABLETTE MIT VERZÖGERTER WIRKSTOFFVERABREICHUNG ZUR ORALEN ANWENDUNG
CORE TABLET FOR CONTROLLED RELEASE OF GLICLAZIDE AFTER ORAL ADMINISTRATION

(30) Priorité: 01.02.1999 FR 9901082
(43) Date de publication de la demande: 31.10.2001
(73) Titulaire: LES LABORATOIRES SERVIER, 92200 Neuilly sur Seine (FR)
(72) Inventeur: HUET DE BAROCHEZ, Bruno, F-45140 Ingre (FR); WUTHRICH, Patrick, F-45000 Orléans (FR); MARTIN, Louis, F-45160 Olivet (FR)
(86) Numéro de dépôt international: PCT/FR1999/002520
(87) Numéro de publication internationale: WO 2000/018373

(56) Documents cités:
- EP-A- 0 468 392
- WO-A-95/11006
- WO-A-99/18932
- US-A- 3 983 249
- US-A- 4 056 623

## Description

La présente invention a pour objet un comprimé matriciel permettant la libération prolongée du gliclazide, insensible aux variations de pH du milieu de dissolution, et assurant des taux sanguins réguliers et constants après absorption de la forme galénique par voie orale.

Le gliclazide, composé de formule (I): 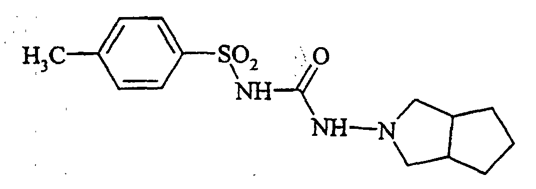 est un dérivé de sulphonylurée à propriété antidiabétique aux doses habituellement administrées chez l'homme.

Le gliclazide était jusqu'à présent administré par voie orale sous forme de comprimés dosés à 80 mg. La prescription moyenne habituelle est de deux comprimés par jour en deux prises, mais peut varier de 1 à 4 comprimés par jour en plusieurs prises selon la sévérité du diabète.

L'un des buts de la présente invention était d'obtenir une forme orale administrable en une seule prise journalière. Ceci permet d'une part une utilisation plus aisée pour le patient et d'autre part une meilleure compliance au traitement.

Un autre but de l'invention était que cette forme orale soit à libération prolongée. En effet, une forme à libération immédiate peut conduire, chez certains sujets, à l'obtention de concentrations sanguines importantes et de courte durée. Une forme à libération prolongée permet d'éviter ces pics sanguins et d'obtenir une concentration sanguine régulière chez l'homme. Ceci permet de réduire les effets indésirables pouvant éventuellement survenir par "effet de pic" accompagnés de troubles de type hydroélectrolytiques et métaboliques reliés aux variations des taux plasmatiques du principe actif.

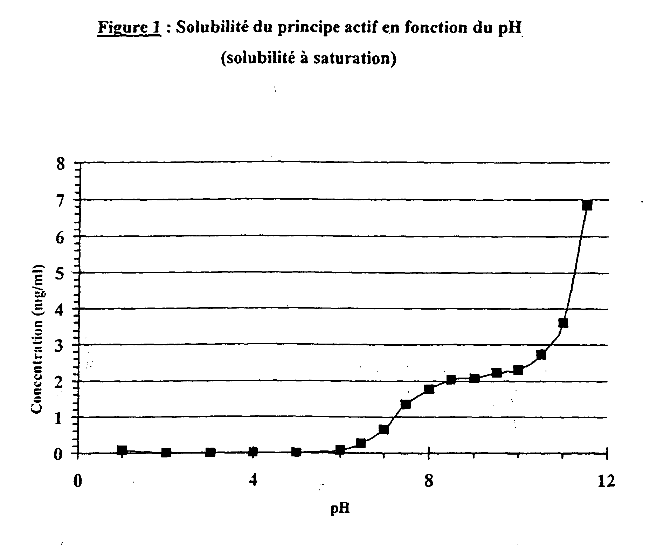

Le but principal de l'invention était d'obtenir une forme orale dont la vitesse de libération du principe actif soit contrôlée et reproductible. En effet, la forme actuelle présente une forte variabilité de la dissolution du principe actif en fonction du pH. Cette particularité liée au gliclazide lui-même induit des problèmes d'absorption du principe actif. Ce phénomène de solubilité du principe actif variant en fonction du pH est illustré par la figure 1 (annexe). La solubilité est très faible aux pH acides et croît avec l'augmentation du pH.

Il était donc important, pour ce principe actif, de mettre au point une nouvelle forme galénique qui permette une libération du gliclazide indépendante du pH du milieu de dissolution.

Plus particulièrement, la présente invention décrit une matrice hydrophile, administrable par voie orale, qui permet une libération prolongée et contrôlée du principe actif, le gliclazide, sans influence du pH sur la cinétique de dissolution in vitro de ladite matrice.

Cette forme à libération prolongée de gliclazide, utile pour le traitement du diabète, permet d'assurer des taux plasmatiques plus réguliers ainsi que des variations Cₘₐₓ-Cₘᵢₙ plus faibles. La vitesse de libération doit être reproductible et corrélée avec les concentrations sanguines observées après administration.

Parmi les mécanismes pouvant être invoqués pour le contrôle de la diffusion d'un principe actif soluble, on peut en retenir un principal, qui est la diffusion du principe actif au travers d'un gel formé après gonflement d'un polymère hydrophile mis au contact du liquide de dissolution (in vitro), ou du liquide gastro-intestinal (in vivo).

De nombreux polymères ont été décrits comme pouvant permettre la formation de ce gel. Les principaux sont les dérivés de la cellulose, en particulier les éthers de cellulose tels que l'hydroxypropylcellulose, l'hydroxyéthylcellulose, la méthylcellulose, l'hydroxypropylméthylcellulose, et parmi les différentes qualités commerciales de ces éthers celles présentant des viscosités assez élevées. Il faut noter que les systèmes décrits ne permettent pas théoriquement d'atteindre un ordre zéro dans l'équation de la cinétique de libération.

Les procédés de fabrication couramment utilisés pour la fabrication de tels comprimés matriciels sont soit la compression directe, après mélange des différents excipients et du ou des principes actifs, soit la granulation humide.

Le comprimé matriciel de gliclazide décrit dans la présente invention associe de façon originale au moins un polymère dérivé de la cellulose et un sirop de glucose (hydrolysat d'amidon de maïs), permettant d'obtenir une libération du principe actif parfaitement prolongée et contrôlée.

La libération contrôlée est linéaire pendant plus de huit heures et est telle que 50 % de la quantité totale de gliclazide est libérée entre 4 et 6 heures. D'autre part, le comprimé matriciel selon l'invention permet d'obtenir une libération prolongée de gliclazide conduisant à des taux sanguins chez l'homme compris entre 400 et 700 ng/ml, 12 heures au plus après administration unique, par voie orale, d'un comprimé dosé à 30 mg de gliclazide et à des taux sanguins compris entre 250 et 1000 ng/ml après administration journalière d'un comprimé dosé à 30 mg de gliclazide.

La posologie unitaire peut varier selon l'âge et le poids du patient, la nature et la sévérité du diabète. D'une façon générale, elle s'échelonne entre 30 et 120 mg, en une seule prise, pour un traitement journalier. Le pourcentage de gliclazide, au sein du comprimé matriciel, est compris entre 12 et 40 % de la masse totale du comprimé. Selon une variante avantageuse de l'invention, ledit comprimé est dosé à 60 mg de gliclazide. Un mode de réalisation particulièrement préféré de l'invention est l'obtention de comprimés dosés à 30 mg de gliclazide. Dans ces cas très avantageux de l'invention, la posologie unitaire qui s'échelonne entre 30 et 120 mg, en une seule prise journalière, correspond à l'absorption de 1 à 4 comprimés dosés à 30 mg ou bien de 1 à 2 comprimés dosés à 60 mg. Le comprimé matriciel, tel que décrit par la Demanderesse, permet d'une part d'avoir une forme orale administrable en une seule prise journalière et d'autre part, de façon surprenante et particulièrement avantageuse, de diminuer la quantité de principe actif au sein de chaque comprimé, sans modifications ou. altérations des concentrations plasmatiques en gliclazide. La formulation existante jusqu'à présent était dosée à 80 mg de gliclazide.

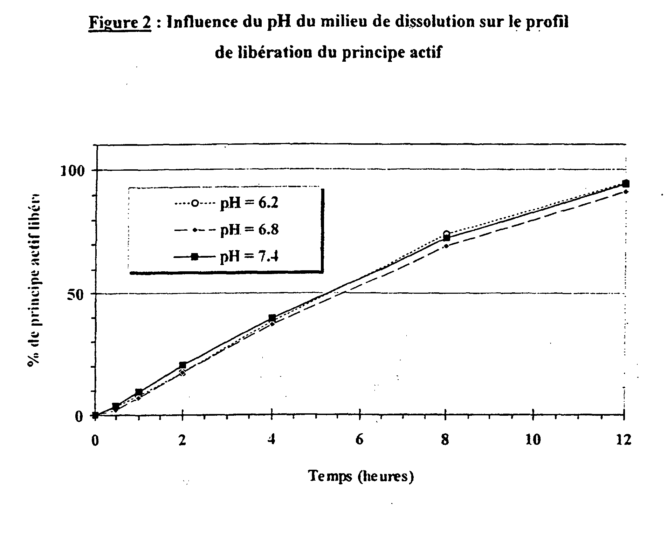

L'association spécifique des composés décrits précédemment permet de plus, de façon surprenante, de ne pas avoir d'influence du pH sur la cinétique de dissolution in vitro de ladite matrice bien que la solubilité du principe actif varie en fonction de ce même pH. Ce point est illustré par la figure 2 (annexe) qui montre que la matrice telle que composée est insensible aux variations de pH dans une gamme de 6,2 à 7,4, pH du milieu intestinal. Ainsi, dans une gamme de pH allant de 6 à 8 et correspondant à la zone ascendante de la courbe décrite dans la figure 1 (annexe), on constate que le profil de libération du principe actif, entre 0 et 12 heures, est identique quelque soit le pH du milieu de dissolution du comprimé matriciel renfermant ledit principe actif.

Ainsi, par la combinaison caractéristique d'au moins un polymère dérivé de la cellulose et un sirop de glucose, la Demanderesse crée une matrice hydrophile innovante tant dans sa composition que dans sa fonction, puisqu'elle permet notamment que le principe actif qu'elle renferme, le gliclazide, soit libéré de façon prolongée et contrôlée, quelles que soient les conditions de pH du milieu de dissolution.

Le polymère dérivé de la cellulose utilisé au sein de cette matrice hydrophile, est un éther de cellulose de haute viscosité. De façon avantageuse, l'éther de cellulose est une hydroxypropylméthylcellulose, et préférentiellement, un mélange de deux hydroxypropylméthylcelluloses de viscosité différente. L'autre composé intervenant dans la composition de ladite matrice est un sirop de glucose, et de façon avantageuse, on utilise de la maltodextrine qui correspond à un sirop de glucose dont le degré de dextrose équivalent (DE) est compris entre 1 et 20. L'association de ces deux catégories de composés permet, d'une part, d'obtenir une formulation dans laquelle le profil de libération du principe actif est insensible aux variations de pH du milieu de dissolution et, d'autre part, d'obtenir un parfait contrôle de la cinétique de libération. Le pourcentage de polymère dérivé de la cellulose est compris entre 10 et 40 % de la masse totale du comprimé, et selon une variante particulièrement avantageuse, entre 16 et 26 % de la masse totale du comprimé. Le pourcentage du sirop de glucose est compris entre 2 et 20 % de la masse totale du comprimé, et de façon préférentielle entre 4 et 10 % de la masse totale du comprimé.

Différents excipients peuvent également être ajoutés afin de finaliser la formulation. Parmi les diluants classiquement utilisés, on utilise de façon préférentielle de l'hydrogénophosphate de calcium dihydraté qui permet d'obtenir une meilleure fluidité des granulés et une meilleure compressibilité de ces derniers. De plus, l'hydrogénophosphate de calcium dihydraté peut ralentir les cinétiques de dissolution, cette caractéristique permettant d'utiliser des quantités d'hydroxypropylméthylcellulose moins importantes pour contrôler le profil de dissolution du principe actif Le pourcentage d'hydrogénophosphate de calcium dihydraté est compris entre 35 et 75 % de la masse totale du comprimé et de façon préférentielle entre 45 et 60 % de la masse totale du comprimé. Parmi les lubrifiants, on peut citer à titre indicatif le stéarate de magnésium, l'acide stéarique, le béhénate de glycérol ou le benzoate de sodium, et parmi les agents d'écoulement on utilise de préférence la silice colloïdale anhydre.

La présente invention concerne également la préparation de ce comprimé matriciel. Une granulation humide est effectuée par mélange du principe actif, du sirop de glucose et de l'hydrogénophosphate de calcium dihydraté, puis mouillage de ce mélange. Cette première étape permet de créer autour du principe actif un environnement hydrophile favorable à sa bonne dissolution, et également d'obtenir un dosage unitaire le plus régulier possible. Dans une seconde étape, le granulé obtenu précédemment est mélangé avec l'éther de cellulose. Si on le souhaite l'éther de cellulose peut être granulé directement avec le principe actif dans la première étape. Puis le mélange est lubrifié par addition de la silice colloïdale et du stéarate de magnésium. Le composé final lubrifié est alors soumis à une compression.

Les exemples suivants illustrent l'invention mais ne la limitent en aucune façon.

La préparation des comprimés, à libération prolongée, administrables par voie orale, est réalisée selon le procédé de fabrication suivant :

### STADE A :

Mélange du gliclazide, de la maltodextrine et de l'hydrogénophosphate de calcium dihydraté, puis mouillage de ce mélange au moyen d'eau purifiée. La masse humide préparée est ensuite granulée, séchée puis calibrée, de manière à obtenir un granulé dont les caractéristiques physiques permettent un bon remplissage des matrices d'une machine à comprimer rapide.

### STADE B :

Mélange du granulé obtenu au stade A avec l'hydroxypropylméthylcellulose.

### STADE C :

Lubrification du mélange obtenu au stade B avec de la silice colloïdale et du stéarate de magnésium.

### STADE D :

Compression du mélange lubrifié obtenu au stade C sur machine à comprimé rotative, de manière à obtenir des comprimés ayant une dureté mesurée par écrasement diamétral d'environ 6 à 10 daN.

### EXEMPLE 1 :

L'exemple 1 montre l'influence de la maltodextrine sur la cinétique de libération in vitro. La quantité de maltodextrine varie de 7,5 à 15 mg par comprimé, représentant ainsi de 4 à 10 % du poids total du comprimé. La quantité d'hydroxypropylméthylcellulose reste constante et la quantité de diluant, l'hydrogénophosphate de calcium dihydraté, est ajustée de façon à obtenir des comprimés de poids constant égal à 160 mg. La fabrication est réalisée suivant le procédé opératoire décrit dans les stades A à D.

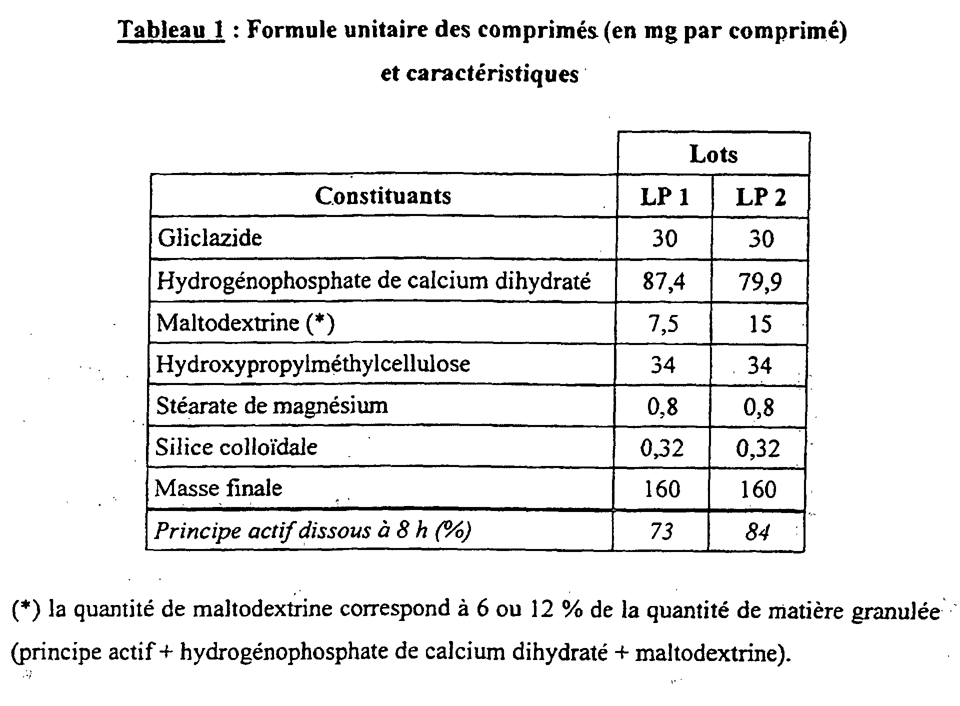

La figure 3 représente les courbes de cinétique de dissolution pour les deux formulations utilisées.

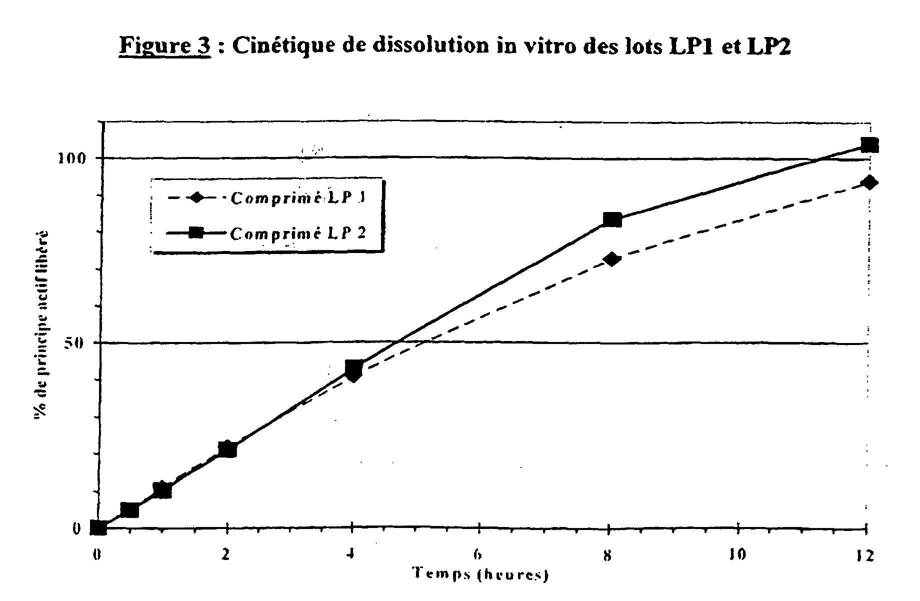

La quantité de maltodextrine, à poids d'hydroxypropylméthylcellulose constant influence la libération du principe actif pour un temps supérieur à 4 heures. En augmentant la quantité de maltodextrine, on linéarise la courbe de dissolution.

### EXEMPLE 2 :

L'exemple 2 montre l'influence de l'hydroxypropylméthylcellulose sur la cinétique de libération in vitro. La quantité d'hydroxypropylméthylcellulose varie de 26 à 42 mg, représentant ainsi de 16 à 26 % du poids total du comprimé.
La fabrication est réalisée suivant le procédé opératoire décrit dans les stades A à D.

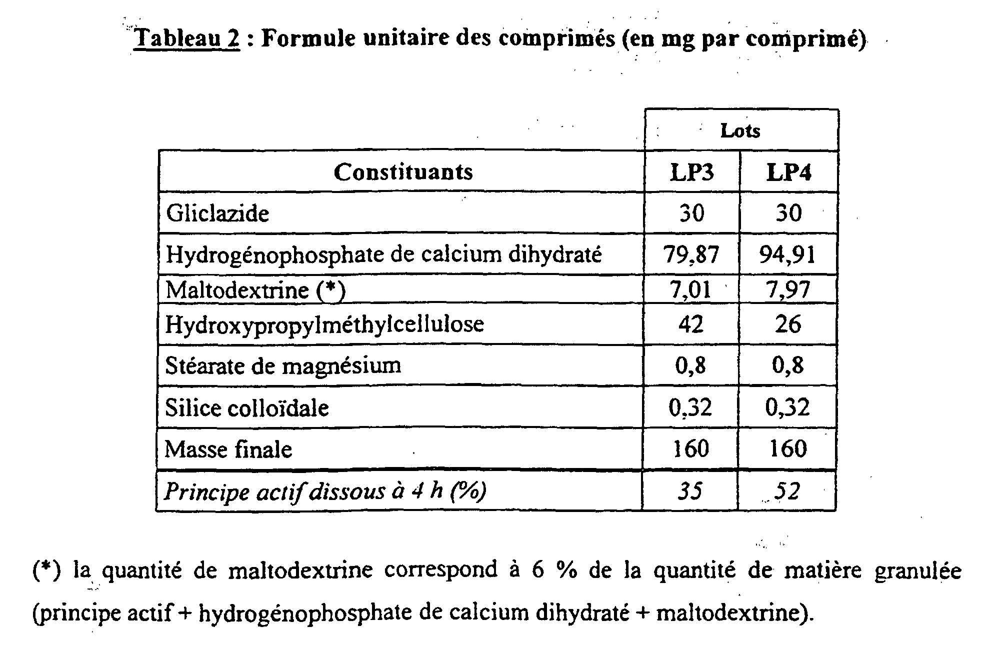

La figure 4 représente les courbes de cinétique de dissolution pour les deux formulations utilisées :

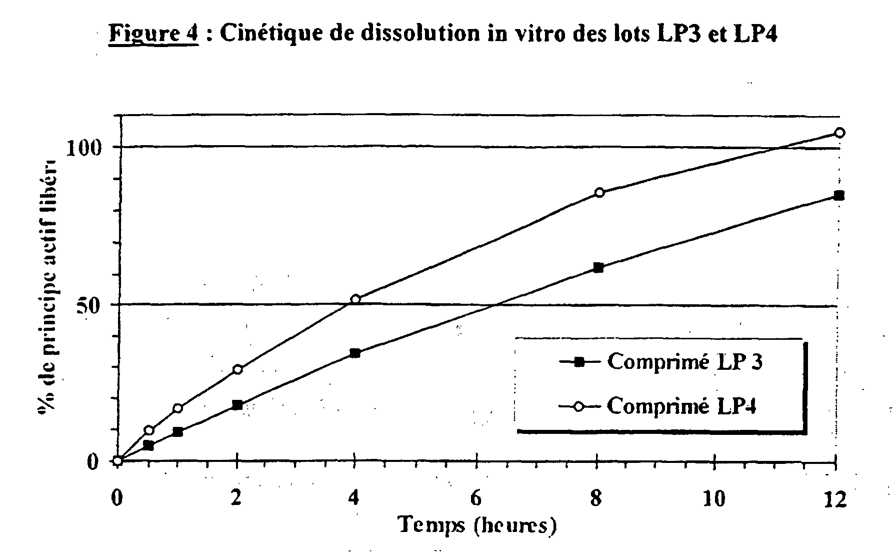

La quantité d'hydroxyproplyméthyicellulose au sein de la matrice hydrophile influence fortement la libération du principe actif.

### EXEMPLE 3 :

L'exemple 3 montre l'influence de la qualité de l'hydroxypropylméthylcellulose utilisée sur la cinétique de libération in vitro. Dans chacun des lots, la masse totale d'hydroxypropylméthylcellulose est constante et on fait varier la quantité relative de chacune des hydroxypropylméthylcelluloses de viscosité différente, permettant ainsi d'obtenir un lot à dissolution lente (LP5) et un lot à dissolution rapide (LP7) par rapport au lot de référence (LP6).

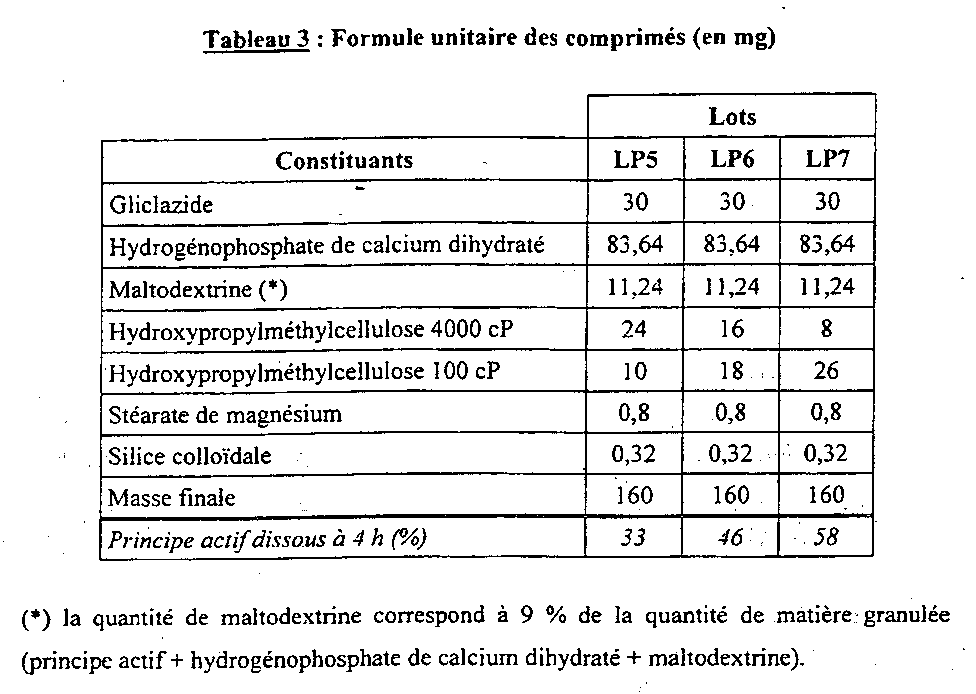

La figure 5 représente les courbes de cinétique de dissolution pour les trois formulations utilisées :

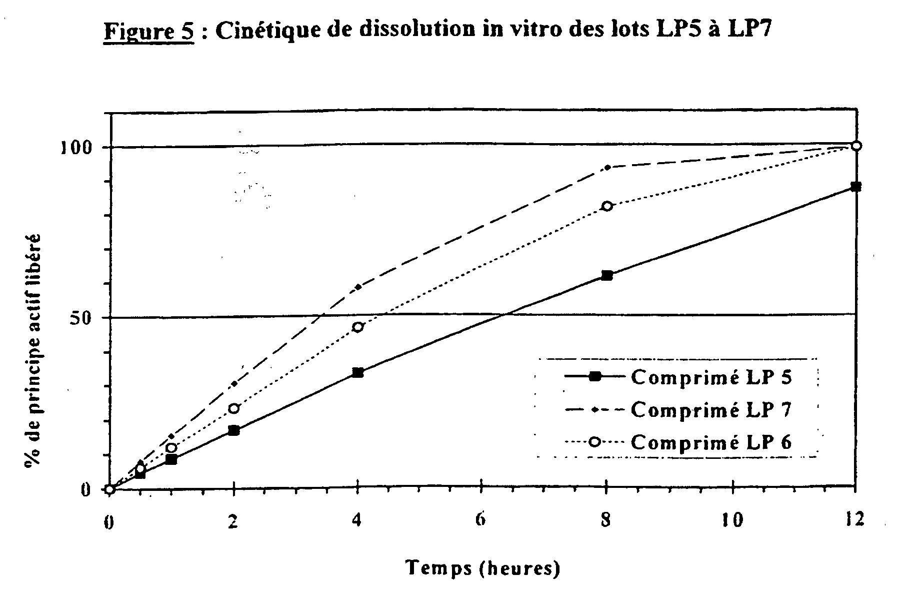

Ces courbes montrent nettement que non seulement la cinétique de dissolution du principe actif est influencée par la quantité globale d'hydroxypropylméthylcellulose utilisée au sein de la matrice hydrophile, mais également par la qualité de l'hydroxypropylméthylcellulose employée.

La cinétique plasmatique de gliclazide est mesurée chez 12 sujets après administration unique de comprimé LP6. La concentration plasmatique moyenne est donnée dans la figure 6.

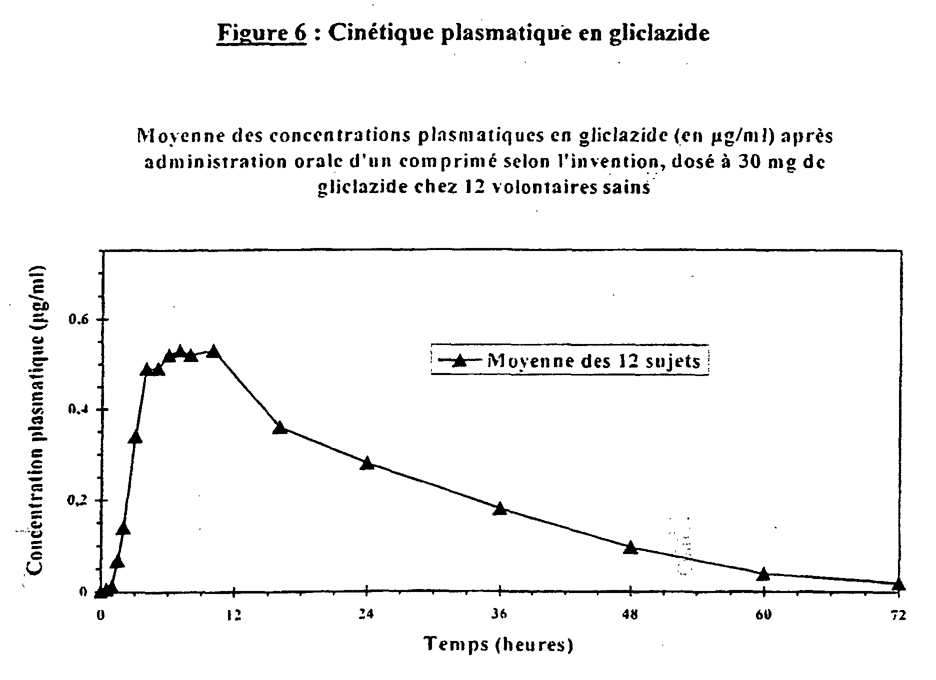

Cette courbe montre un profil de dissolution de type matriciel (libération continue du principe actif), avec une cinétique plasmatique monophasique.

### EXEMPLE 4 :

L'exemple 4 montre que la cinétique de libération in vitro, d'un comprimé dosé à 60 mg, est similaire à celle d'un comprimé dosé à 30 mg (lot LP6) pour des comprimés matriciels dosés de façon identique en hydroxypropylméthylcellulose et en maltodextrine..

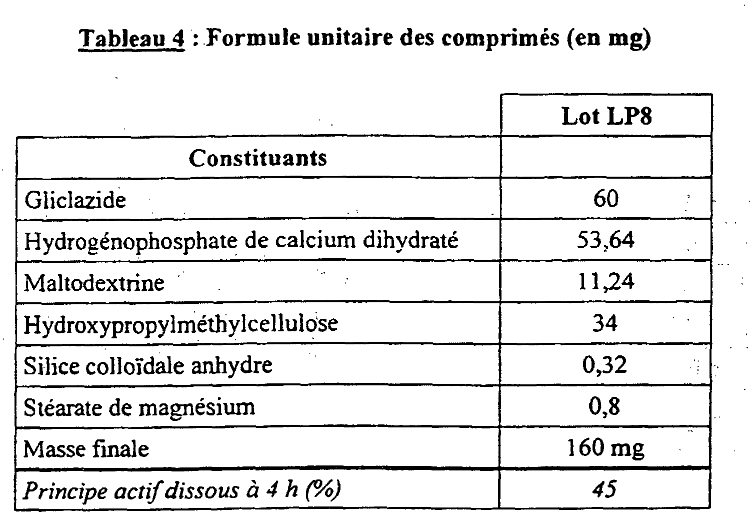

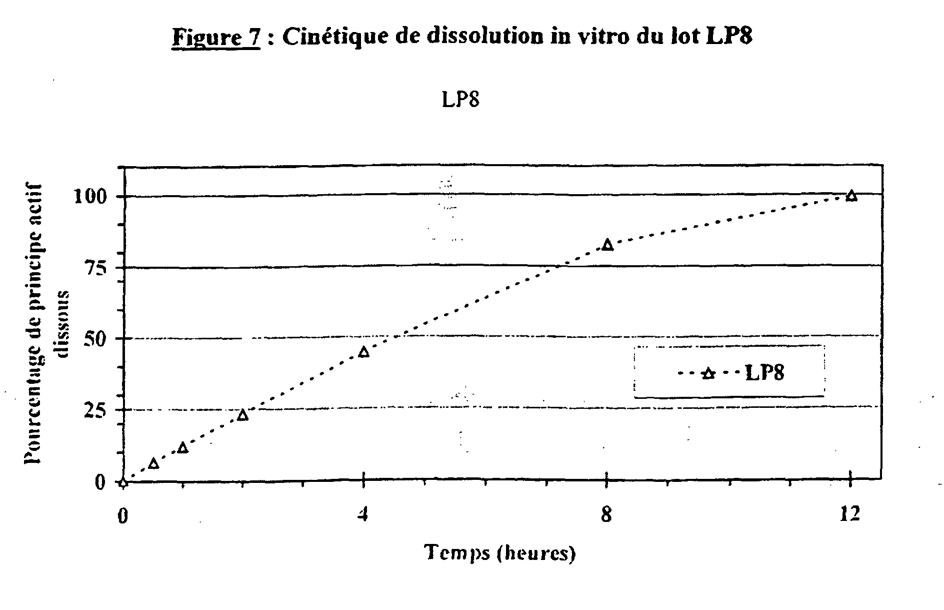

## Revendications

1. Comprimé matriciel pour la libération prolongée de gliclazide **caractérisé en ce qu'**il comporte au moins l'association d'un polymère dérivé de la cellulose et d'un sirop de glucose, ladite association permettant le contrôle de la libération prolongée de gliclazide et l'insensibilité aux variations de pH de la cinétique de dissolution du gliclazide.

2. Comprimé matriciel de gliclazide selon la revendication 1 **caractérisé en ce que** le polymère dérivé de la cellulose est constitué d'au moins une hydroxypropylméthylcellulose.

3. Comprimé matriciel de gliclazide selon l'une quelconque des revendications. 1 et 2 **caractérisé en ce que** le polymère dérivé de la cellulose est constitué d'un mélange de deux hydroxypropylméthylcellulose de viscosité différente.

4. Comprimé matriciel de gliclazide selon l'une quelconque des revendications 1 à 3 **caractérisé en ce que** le polymère dérivé de la cellulose est constitué d'un mélange d'hydroxypropylméthylcellulose de viscosité 4000 cP et d'hydroxypropylméthylcellulose de viscosité 100 cP.

5. Comprimé matriciel de gliclazide selon la revendication 1 **caractérisé en ce que** le sirop de glucose est constitué par de la maltodextrine.

6. Comprimé matriciel de gliclazide selon l'une quelconque des revendications 1 à 4 **caractérisé en ce que** le pourcentage de polymère dérivé de la cellulose est compris entre 10 et 40 % de la masse totale dudit comprimé.

7. Comprimé matriciel de gliclazide selon l'une quelconque des revendications 1, 2, 3, 4 et 6 **caractérisé en ce que** le pourcentage de polymère dérivé de la cellulose est compris entre 16 et 26 % de la masse totale dudit comprimé.

8. Comprimé matriciel de gliclazide selon l'une quelconque des revendications 1 et 5 **caractérisé en ce que** le pourcentage de sirop de glucose est compris entre 2 et 20 % de la masse totale dudit comprimé.

9. Comprimé matriciel de gliclazide selon l'une quelconque des revendications 1, 5 et 8 **caractérisé en ce que** le pourcentage de sirop de glucose est compris entre 4 et 10 % de la masse totale dudit comprimé.

10. Comprimé matriciel de gliclazide selon la revendication 1 **caractérisé en ce que** l'on utilise de l'hydrogénophosphate de calcium dihydraté comme diluant.

11. Comprimé matriciel de gliclazide selon l'une quelconque des revendications 1 et 10 **caractérisé en ce que** le pourcentage de diluant est compris entre 35 et 75 % de la masse totale dudit comprimé.

12. Comprimé matriciel de gliclazide selon l'une quelconque des revendications 1, 10 et 11 **caractérisé en ce que** le pourcentage de diluant est compris entre 45 et 60 % de la masse totale dudit comprimé.

13. Comprimé matriciel de gliclazide selon la revendication 1 **caractérisé en ce que** la quantité de gliclazide soit comprise entre 12 et 40 % de la masse totale dudit comprimé.

14. Comprimé matriciel de gliclazide selon l'une quelconque des revendications 1 et 13 **caractérisé en ce qu'**il contient une quantité totale de gliclazide de 30 mg.

15. Comprimé matriciel de gliclazide selon l'une quelconque des revendications 1 et 13 **caractérisé en ce qu'**il contient une quantité totale de gliclazide de 60 mg.

16. Comprimé matriciel de gliclazide selon la revendication 1 **caractérisé en ce que** les pourcentages du polymère dérivé de la cellulose et du sirop de glucose permettent un profil de libération constant du gliclazide pour un pH du milieu de dissolution variant de 6 à 8.

17. Comprimé matriciel de gliclazide selon la revendication 1 **caractérisé en ce que** les pourcentages du polymère dérivé de la cellulose et du sirop de glucose permettent la libération de 50 % de la quantité totale de gliclazide en un temps compris entre 4 et 6 heures.

18. Comprimé matriciel de gliclazide selon la revendication 1 **caractérisé en ce que** les pourcentages de polymère dérivé de la cellulose et de sirop de glucose permettent une libération prolongée de gliclazide conduisant à des taux sanguins chez l'homme compris entre 400 et 700 ng/ml, 12 heures au plus après administration unique du comprimé par voie orale.

19. Procédé de préparation d'un comprimé matriciel selon la revendication 1 **caractérisé en ce que** l'on utilise à la fois une technique de granulation humide et de compression directe comprenant les étapes suivantes :
***STADE A :***
Mélange du gliclazide, de la maltodextrine et de l'hydrogénophosphate de calcium dihydraté, puis mouillage de ce mélange au moyen d'eau purifiée. La masse humide préparée est ensuite granulée, séchée puis calibrée, de manière à obtenir un granulé dont les caractéristiques physiques permettent un bon remplissage des matrices d'une machine à comprimer rapide.
***STADE B :***
Mélange du granulé obtenu au stade A avec l'hydroxypropylméthylcellulose.
***STADE C :***
Lubrification du mélange obtenu au stade B avec de la silice colloïdale et du stéarate de magnésium.
***STADE D :***
Compression du mélange lubrifié obtenu au stade C sur machine à comprimé rotative, de manière à obtenir des comprimés ayant une dureté mesurée par écrasement diamétral d'environ 6 à 10 daN.

20. Comprimé matriciel de gliclazide selon la revendication 1 utile dans le traitement du diabète.

## Patentansprüche

1. Matrixtablette für die verlängerte Freisetzung von Gliclazid, **dadurch gekennzeichnet, daß** sie mindestens die Kombination aus einem von Cellulose abgeleiteten Polymeren und einem Glucosesirup umfaßt, welche Kombination die Steuerung der verlängerten Freisetzung von Gliclazid und die Unempfindlichkeit der Kinetik der Auflösung von Gliclazid gegenüber pH-Änderungen ermöglicht.

2. Gliclazid-Matrixtablette nach Anspruch 1, **dadurch gekennzeichnet, daß** das von Cellulose abgeleitete Polymere aus mindestens einer Hydroxypropylmethylcellulose gebildet ist.

3. Gliclazid-Matrixtablette nach einem der Ansprüche 1 und 2, **dadurch gekennzeichnet, daß** das von Cellulose abgeleitete Polymere aus einer Mischung aus zwei Hydroxypropylmethylcellulosen mit unterschiedlicher Viskosität gebildet ist.

4. Gliclazid-Matrixtablette nach einem der Ansprüche 1 bis 3, **dadurch gekennzeichnet, daß** das von Cellulose abgeleitete Polymere aus einer Mischung aus Hydroxypropylmethylcellulose und einer Viskosität von 4000 cP und Hydroxypropylmethylcellulose mit einer Viskosität von 100 cP gebildet ist.

5. Gliclazid-Matrixtablette nach Anspruch 1, **dadurch gekennzeichnet, daß** der Glucosesirup durch Maltodextrin gebildet ist.

6. Gliclazid-Matrixtablette nach einem der Ansprüche 1 bis 4, **dadurch gekennzeichnet, daß** der Prozentsatz des von Cellulose abgeleiteten Polymeren zwischen 10 und 40% der Gesamtmasse der Tablette beträgt.

7. Gliclazid-Matrixtablette nach einem der Ansprüche 1, 2, 3, 4 und 6, **dadurch gekennzeichnet, daß** der Prozentsatz des von Cellulose abgeleiteten Polymeren zwischen 16 und 26% der Gesamtmasse der Tablette beträgt.

8. Gliclazid-Matrixtablette nach einem der Ansprüche 1 und 5, **dadurch gekennzeichnet, daß** der Prozentsatz an Glucosesirup zwischen 2 und 20% der Gesamtmasse der Tablette beträgt.

9. Gliclazid-Matrixtablette nach einem der Ansprüche 1, 5 und 8, **dadurch gekennzeichnet, daß** der Prozentsatz des Glucosesirups zwischen 4 und 10% der Gesamtmasse der Tablette beträgt.

10. Gliclazid-Matrixtablette nach Anspruch 1, **dadurch gekennzeichnet, daß** sie als Verdünnungsmittel Calciumhydrogenphosphat-Dihydrat enthält.

11. Gliclazid-Matrixtablette nach einem der Ansprüche 1 und 10, **dadurch gekennzeichnet, daß** der Prozentsatz des Verdünnungsmittels zwischen 35 und 75% der Gesamtmasse der Tablette beträgt.

12. Gliclazid-Matrixtablette nach einem der Ansprüche 1, 10 und 11, **dadurch gekennzeichnet, daß** der Prozentsatz des Verdünnungsmittels zwischen 45 und 60% der Gesamtmasse der Tablette beträgt.

13. Gliclazid-Matrixtablette nach Anspruch 1, **dadurch gekennzeichnet, daß** die Gliclazid-Menge zwischen 12 und 40% der Gesamtmasse der Tablette beträgt.

14. Gliclazid-Matrixtablette nach einem der Ansprüche 1 und 13, **dadurch gekennzeichnet, daß** sie eine Gesamtmenge von 30 mg Gliclazid enthält.

15. Gliclazid-Matrixtablette nach einem der Ansprüche 1 und 13, **dadurch gekennzeichnet, daß** sie eine Gesamtmenge von 60 mg Gliclazid enthält.

16. Gliclazid-Matrixtablette nach Anspruch 1, **dadurch gekennzeichnet, daß** die Prozentsätze an von Cellulose abgeleiteten Polymeren und Glucosesirup bei einem pH-Wert des Auflösungsmediums von 6 bis 8 ein konstantes Gliclazid-Freisetzungsprofil ermöglichen.

17. Gliclazid-Matrixtablette nach Anspruch 1, **dadurch gekennzeichnet, daß** die Prozentsätze des von Cellulose abgeleiteten Polymeren und des Glucosesirups die Freisetzung von 50% der Gliclazid-Gesamtmenge im Verlaufe eines Zeitraums zwischen 4 und 6 Stunden ermöglichen.

18. Gliclazid-Matrixtablette nach Anspruch 1, **dadurch gekennzeichnet, daß** die Prozentsätze des von Cellulose abgeleiteten Polymeren und des Glucosesirups eine verlängerte Freisetzung von Gliclazid ermöglichen, welche 12 Stunden nach der einmaligen Verabreichung der Tablette auf oralem Wege Blutspiegel beim Menschen zwischen 400 und 700 ng/ml ergeben.

19. Verfahren zur Herstellung einer Matrixtablette nach Anspruch 1, **dadurch gekennzeichnet, daß** man gleichzeitig eine Methode der Feuchtgranulation und des direkten Verpressens anwendet, welche die folgenden Stufen umfaßt:
***STUFE A:***
Vermischen von Gliclazid, Maltodextrin und Natriumhydrogenphosphat und Befeuchten dieser Mischung mit gereinigtem Wasser. Anschließend wird die feuchte Masse granuliert, getrocknet und in der Weise kalibriert, daß man ein Granulat enthält, dessen physikalische Eigenschaften eine gute Füllung der Matrizen einer Schnellpreßvorrichtung ermöglichen.
***STUFE B:***
Vermischen des in der Stufe A erhaltenen Granulats mit Hydroxypropylmethylcellulose.
***STUFE C:***
Gleitmittelbehandlung der in der Stufe B erhaltenen Mischung mit kolloidalem Siliciumdioxid und Magnesiumstearat.
***STUFE D:***
Verpressen der in der Stufe C erhaltenen mit Gleitmittel behandelten Mischung mit einer Rotationstablettenpreßvorrichtung unter Bildung von Tabletten mit einer durch diametrales Zerquetschen gemessenen Härte von etwa 6 bis 10 daN.

20. Gliclazid-Matrixtablette nach Anspruch 1 für die Behandlung von Diabetes.

## Claims

1. Matrix tablet for the prolonged release of gliclazide, **characterised in that** it comprises at least the combination of a cellulose polymer compound and a glucose syrup, that combination enabling control of the prolonged release of gliclazide and enabling insensitivity of the dissolution kinetics of gliclazide to variations in pH.

2. Gliclazide matrix tablet according to claim 1, **characterised in that** the cellulose polymer compound comprises at least one hydroxypropyl methylcellulose.

3. Gliclazide matrix tablet according to either claim 1 or claim 2, **characterised in that** the cellulose polymer compound comprises a mixture of two hydroxypropyl methylcelluloses of different viscosity.

4. Gliclazide matrix tablet according to any one of claims 1 to 3, **characterised in that** the cellulose polymer compound comprises a mixture of hydroxypropyl methylcellulose of viscosity 4000 cP and hydroxypropyl methylcellulose of viscosity 100 cP.

5. Gliclazide matrix tablet according to claim 1, **characterised in that** the glucose syrup is maltodextrin.

6. Gliclazide matrix tablet according to any one of claims 1 to 4, **characterised in that** the percentage of cellulose polymer compound is from 10 to 40 % of the total weight of the said tablet.

7. Gliclazide matrix tablet according to any one of claims 1, 2, 3, 4 and 6, **characterised in that** the percentage of cellulose polymer compound is from 16 to 26 % of the total weight of the said tablet.

8. Gliclazide matrix tablet according to either claim 1 or claim 5, **characterised in that** the percentage of glucose syrup is from 2 to 20 % of the total weight of the said tablet.

9. Gliclazide matrix tablet according to any one of claims 1, 5 and 8, **characterised in that** the percentage of glucose syrup is from 4 to 10 % of the total weight of the said tablet.

10. Gliclazide matrix tablet according to claim 1, **characterised in that** calcium hydrogen phosphate dihydrate is used as diluent.

11. Gliclazide matrix tablet according to either claim 1 or claim 10, **characterised in that** the percentage of diluent is from 35 to 75 % of the total weight of the said tablet.

12. Gliclazide matrix tablet according to any one of claims 1, 10 and 11, **characterised in that** the percentage of diluent is from 45 to 60 % of the total weight of the said tablet.

13. Gliclazide matrix tablet according to claim 1, **characterised in that** the amount of gliclazide is from 12 to 40 % of the total weight of the said tablet.

14. Gliclazide matrix tablet according to either claim 1 or claim 13, **characterised in that** it contains a total amount of gliclazide of 30 mg.

15. Gliclazide matrix tablet according to either claim 1 or claim 13, **characterised in that** it contains a total amount of gliclazide of 60 mg.

16. Gliclazide matrix tablet according to claim 1, **characterised in that** the percentages of cellulose polymer compound and glucose syrup make possible a constant gliclazide release profile for a dissolution medium pH ranging from 6 to 8.

17. Gliclazide matrix tablet according to claim 1, **characterised in that** the percentages of cellulose polymer compound and of glucose syrup make possible the release of 50% of the total amount of gliclazide by a time of from 4 to 6 hours.

18. Gliclazide matrix tablet according to claim 1, **characterised in that** the percentages of cellulose polymer compound and of glucose syrup enable prolonged release of gliclazide that results in humans in blood levels of from 400 to 700 ng/ml 12 hours at most after a single administration of the tablet by the oral route.

19. Process for the preparation of a matrix tablet according to claim 1, **characterised in that** there are used both a wet granulation technique and a direct compression technique, comprising the following steps :
***STEP A :***
Mixture of gliclazide, maltodextrin and calcium hydrogen phosphate dihydrate, followed by wetting of that mixture with purified water. The resulting wet mass is then granulated, dried and subsequently classified to obtain a granulate having physical characteristics that enable good filling of the moulds of a rapid-compression machine.
***STEP B :***
Mixture of the granulate obtained in Step A with hydroxypropyl methylcellulose.
***STEP C :***
Lubrication of the mixture obtained in Step B with colloidal silica and magnesium stearate.
***STEP D :***
Compression of the lubricated mixture obtained in Step C using a rotary compression machine to obtain tablets having a hardness, measured by diametric crushing, of about from 6 to 10 daN.

20. Gliclazide matrix tablet according to claim 1 for use in the treatment of diabetes.
